(19) Europäisches Patentamt
European Patent Office
Office européen des brevets

(11) **EP 3 594 963 A1**

(12) **EUROPEAN PATENT APPLICATION**

(43) Date of publication:
**15.01.2020  Bulletin 2020/03**

(51) Int Cl.:
**G16H 50/30** (2018.01)     **A61B 5/00** (2006.01)
**G16H 50/20** (2018.01)     **A61B 5/053** (2006.01)

(21) Application number: **18182894.8**

(22) Date of filing: **11.07.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(72) Inventors:
• **Ouwerkerk, Martin**
  **5656 AE Eindhoven (NL)**
• **Denissen, Adrianus Johannes Maria**
  **5656 AE Eindhoven (NL)**
• **Westerink, Joanne Henriëtte Desirée Monique**
  **5656 AE Eindhoven (NL)**

(71) Applicant: **Koninklijke Philips N.V.**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DEVICE, SYSTEM AND METHOD FOR DETERMINING A STRESS LEVEL OF A USER**

(57)    The present invention relates to a device, system and method for determining a stress level, in particular for determining a psychogenic stress level of a user. The device comprises an interface (11) configured to obtain a skin conductance signal trace (22) of the user; a processing unit (12) configured to process the obtained skin conductance signal trace by: identifying a plurality of skin conductance peaks (50) in the skin conductance signal trace; determining, for each of said skin conductance peaks, a normalized parameter (58) of said skin conductance peak, normalized based on a skin conductance value (52, 53) of the respective skin conductance peak (50); and determining a psychogenic stress level (68) of the user based on said normalized parameters of said skin conductance peaks. The invention further relates to a corresponding wearable device (30) comprising such a device (10).

FIG.1

**Description**

FIELD OF THE INVENTION

The present invention relates to a device, system and method for determining a stress level, in particular for determining a psychogenic stress level of a user. The present invention relates further to a wearable device and to a computer program.

BACKGROUND OF THE INVENTION

Mental stress and emotional stress, often called psychological stress, can compromise health when they are not adequately dealt with. Short periods of psychological stress can cause sleep disturbance, fatigue, headaches, and mood changes. Accumulated psychological stress can cause anxiety, depression, chronic fatigue, digestive problems, autoimmune disease, and cardiovascular disease. Psychological stress, further referred to as (psychogenic) stress, and its related comorbid diseases are responsible for a large proportion of disability worldwide. This finding clearly underlines the need for pro-active reduction of stress by adequate coping to prevent stress to exert its harmful short and long-term effects on the body and mind.

Stress is characterized by a series of bodily responses, including an increased heart rate, increased sweating (at multiple locations of the body) resulting in a rise in skin conductance, and the release of the stress hormone cortisol with a delay of about 20-30 min. These bodily responses facilitate an adequate response towards an actual or perceived stressor, such as a threat, or a surprise, or any other stimulus or event that causes stress. However, too high stress levels can have a negative impact on a person's health.

Skin conductance is a known indicator for measuring a stress level of a person, as described in US 9,962,104 B2, an earlier granted patent of the applicant. In response to stressors, the sympathetic outflow of the autonomous nervous system activates sweat glands, and the skin conductance rises as a result of this activation. However, sweat glands are also activated by thermoregulation. In other words, there are two main causes for sweating: thermoregulation and mental stress. The first type of sweating is also referred to as thermogenic sweating and the second type is referred to as psychogenic sweating. In many cases, users are only interested in a mental stress level, which can be obtained from psychogenic sweating. In this context, physical exercise, such as running, is also considered as thermogenic sweating. Physical exercise may elevate the core body temperature beyond a limit where thermoregulation effects including sweating begin.

When measuring the skin conductance of a person, it is thus difficult to distinguish between thermogenic sweating and psychogenic sweating.

The scientific publication by Machado-Moreira et al., "Thermogenic and psychogenic recruitment of human eccrine sweat glands: Variations between glabrous and non-glabrous skin surfaces", Journal of Thermal Biology, Elsevier, 2017, investigates human eccrine sweat-gland recruitment and secretion rates from the glabrous (volar, i.e., non-hairy) and non-glabrous (dorsal, i.e., hairy) hand surfaces during psychogenic (mental arithmetic) and thermogenic stimuli (mild hyperthermia). It was found that psychogenic sweating dominates for the volar (palmar / glabrous, non-hairy) hand surface whereas thermogenic sweating dominates for the dorsal (non-glabrous, hairy) hand surface.

Accordingly, US 9,962,104 B2 discloses a stress-measuring device and method, wherein the electrodes for skin conductance measurement are arranged at a palmar, non-hairy side of the wrist so that a reliable measurement indicative of psychogenic sweating can be obtained (see e.g. Fig. 3 of US 9,962,104 B2).

Regarding the signal processing, US 9,962,104 B2 discloses a stress-measuring device comprising an input interface for receiving a skin conductance signal indicating the skin conductance of the user, the skin conductance signal over time forming skin conductance trace data. The stress-measuring device further comprises a processing unit for processing the skin conductance trace data, wherein the processing unit is adapted to determine, over at least a portion of the skin conductance trace data, values of a rise time between at least two different points of the skin conductance trace data, to determine a frequency distribution of the rise time values, and to determine the level of stress of the user based on the determined frequency distribution.

SUMMARY OF THE INVENTION

It is an object of the present invention to provide an improved device, system and method as well as a wearable device that enable determining a stress level, in particular a mental stress level, of a user. In particular, it would be desirable to determine a mental stress level in an easy manner and with sufficient reliability. It would also be desirable to reduce the system complexity, improve the wearing comfort of a wearable device and to reduce the overall system cost.

In a first aspect of the present invention a device for determining a (psychogenic) stress level of a user is presented that comprises:

- an interface configured to obtain (e.g. to receive or to retrieve) a skin conductance signal trace of the user; and
- a processing unit configured to process the obtained skin conductance signal trace by:
- identifying a plurality of skin conductance peaks in the skin conductance signal trace;
- determining, for each of said skin conductance peaks, a normalized parameter of said skin conductance peak, normalized based on a skin conductance value of the respective skin conductance peak; and
- determining a psychogenic stress level of the user based on said normalized parameters of said skin conductance peaks.

In a further aspect of the present invention a system for determining a stress level of a user is presented, said system comprising:

- a sensor for acquiring a skin conductance signal trace from a user; and
- a device as disclosed herein.

In a further aspect of the present invention a wearable device wearable by a user is presented, the wearable device comprising the system as disclosed herein.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed. It shall be understood that in the context of a computer implemented method the step of obtaining the skin conductance signal trace can refer to receiving or retrieving data descriptive of said skin conductance signal trace from a database or (temporary) data storage or as a stream.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

Skin conductance measurements include contributions from thermogenic sweating and psychogenic sweating. However, would be desirable to separate both contributions efficiently. At first glance, it may seem counter-intuitive to use normalized skin conductance values, because, by normalizing data, information may be lost. However, the inventors have recognized that contributions from thermogenic sweating may act as a scaling factor on contributions from psychogenic sweating. Hence, in order reduce the impact of contributions from thermogenic sweating, it is suggested to normalize a parameter, such as an amplitude or a (rising) edge slope, of the skin conductance peak based on a skin conductance value of said same respective skin conductance peak. Thereby, the impact of contributions from thermogenic sweating can be eliminated or at least reduced, leaving the effects of psychogenic sweating. A mental stress level may thus be determined with improved accuracy, irrespective of thermogenic sweating.

As described above, psychogenic sweating is indicative of a mental stress level. Hence, a psychogenic or mental stress level can be determined with improved reliability even from a skin conductance signal trace also comprising contributions due to thermogenic sweating.

Further advantages of the proposed solution may include that the proposed signal processing can be implemented with low computational effort, in an easy manner but nevertheless with sufficient reliability. Advantages may include that the system complexity for separating contributions from psychogenic and thermogenic sweating may be limited. Further, the proposed solution may enable the acquisition of skin conductance signal traces also at non-glabrous (=hairy) skin surfaces such as at an upper or dorsal side of the wrist. It shall be understood that measurements at other sites such as the ankles or further potentially hairy skin surfaces may also benefit from the solutions proposed herein.

It should be noted that in order to determine psychogenic sweating, the aforementioned publication by Machado-Moreira et al. teaches the different approach of measuring psychogenic sweating at a glabrous (non-hairy) volar hand surface. However, this approach does not seem to be convenient in everyday situations, in particular for long-term monitoring. To the contrary, the method described therein may be considered as cumbersome and obtrusive. In view of the teachings of the aforementioned publication by Machado-Moreira, one may gain the impression that stress responses nowadays cannot be measured accurately at the upper side of the wrist because the sensor data contains strong signals linked to thermoregulation. This is because the upper side of the wrist is non-glabrous (hairy) skin. For accurate stress response measurements, the palm of the hand is used, which is glabrous (non-hairy) skin. In daily life positioning a sensor on the palm of the hand is undesirable, since it hampers many activities.

The proposed invention may provide a solution to overcome or at least reduce one or more of these issues.

It should be noted that while the proposed solution may be particularly useful if one wants to measure psychogenic skin conductance changes on the outside (dorsal side) of the wrist, the normalization approach can also be useful on the inside of the wrist. Even at the inside of the wrist there can be an impact of thermogenic sweating and even though the impact may be lower than on the outside of the wrist, a measurement at the inside of the wrist may benefit from the solution provided herein.

Moreover, a measurement at an upper or dorsal side of the wrist may facilitate implementation of a wrist-worn wearable device since a sensor for skin conductance measurement, in particular the electrodes for skin conductance measurement, may be implemented at a lower side of a wearable device housing and there may not be a need to provide a dedicated (expensive) wrist band comprising skin conductance measurement electrodes for measurement a glabrous lower side of the wrist.

A further advantage of using a wearable device comprising skin conductance measurement electrodes at a lower side of the housing, wherein the electrodes are adapted to, in an as worn orientation, contact an upper side of the wrist can be that the electrodes are pressed onto the skin of the user by gravity. Hence, an improved physical and electrical contact for accurate measurement may be provided. A measurement at an upper side of the wrist can also be beneficial because at the underside there are many muscle tendons, which flex when the fingers are moving. This may causes instabilities in the contact area. It has been found, even though the upper side may be influenced by thermal sweating, a measurement at the top area can be more stable in this aspect.

It should further be noted that the solution proposed herein, to a certain extent, differs from the approach normally used in the practice of extracting data from skin conductance trace, as for example described in the standard textbook "Techniques in Psychophysiology", John Wiley & Sons Ltd. 1980, see in particular Chapter 1 on "Electrodermal Activity" as well as in "Electrodermal activity", 2nd edition, Springer Verlag 2012. The standard method for measuring the amplitude of the skin conductance response takes the skin conductance level at the top and deducts the skin conductance level at the onset, thus yielding a number with the same dimension as the skin conductance (usually micro Siemens). This (conventional) amplitude can be referred to as the absolute skin conductance response amplitude, or $SCR_{absolute\_amplitude}$.

The terminology used herein and techniques regarding skin conductance measurements may be described as indicated in the standard textbook "Techniques in Psychophysiology", John Wiley & Sons Ltd. 1980, see in particular Chapter 1 on "Electrodermal Activity" as well as in "Electrodermal activity", 2nd edition, Springer Verlag 2012, the contents of which are incorporated herein by reference, respectively.

As used herein, obtaining a skin conductance signal trace can refer to receiving or retrieving the skin conductance signal trace. The skin conductance signal trace can be acquired from a skin of the user using electrodes adapted to contact the skin of the subject. The skin conductance signal trace can be indicative (or descriptive) of a skin conductance of the user over time.

A skin conductance peak (SCP) can refer to a portion of the skin conductance signal trace comprising a skin conductance peak. A skin conductance peak can also refer to a skin conductance response (SCR). A skin conductance peak or skin conductance response can be identified using known techniques. For example, a first derivative of the skin conductance signal trace can be evaluated for zero-crossings. Identifying skin conductance responses in the skin conductance signal can comprise identifying local maxima in the skin conductance signal trace and identifying said skin conductance responses based on said local maxima. It shall be understood that one or more suitable pre-processing steps such as low-pass filtering may be applied before performing as part of or in the step of identifying a plurality of skin conductance peaks (50) in the skin conductance signal trace. For example, a skin conductance signal trace acquired at a sampling rate of for example 160Hz may be polluted with 50Hz mains noise. This can be filtered out with a suitable low-pass filter before the peak detection can take place.

A normalized amplitude of a skin conductance peak, also referred to as normalized or relative skin conductance peak amplitude, can represent a normalized height of the respective skin conductance peak. It should be noted that different ways of normalization can be applied. For example an amplitude of the skin conductance peak may be divided by an average skin conductance level of that (section of) the trace or a section of the trace neighboring or surrounding the respective skin conductance peak. The skin conductance peak may be normalized based on a normalization factor, wherein the normalization factor is based on a section of the skin conductance signal trace neighboring or surrounding the respective skin conductance peak.

A stress level of the user can be determined using known methods for determining a stress level such as, for example, evaluating a sum of rising edge amplitudes. However, according to the present disclosure, the evaluated parameters may further have undergone normalization. In an embodiment, determining a psychogenic stress level based on said normalized skin conductance response amplitudes may thus comprise determining a sum of said normalized skin conductance parameters, such as a normalized amplitude or steepness of the respective skin conductance peaks, over a predetermined time and comparing said sum with a predetermined threshold. In an embodiment, only the positive first derivative (rising edge) may be used and negative values may be discarded, e.g. by setting them to zero.

In an aspect, the solution proposed herein may be seen as an additional intermediate or pre-processing step between one or more known signal acquisition steps and one or more known further processing steps.

In an embodiment, for each of said skin conductance peaks, determining the normalized parameter can comprise scaling (or normalizing) a first value of the skin conductance signal trace at the respective skin conductance peak based on or by a second value of the skin conductance signal trace at the respective skin conductance peak. The normalization may be performed on a peak-to-peak or SCR-to-SCR basis. Hence, a local normalization can be provided by normalizing each of the plurality of skin conductance response peaks individually. The normalization may be based on the underlying level or a value of the skin conductance trace at each respective peak individually.

In an embodiment, the processing unit can be adapted such that, for each of said skin conductance peaks, said normalized parameter is a normalized amplitude of the respective skin conductance peak. It will be understood that this may also include that the normalized parameter is indicative of or determined based on a normalized amplitude of the respective skin conductance peak. Hence, instead of performing an absolute amplitude measurement of the skin conductance, e.g. in $\mu$S (microSiemens), e.g. by taking difference between two values of the skin conductance peak, it is suggested that a normalized amplitude of the respective skin conductance peak is determined. By determining a normalized amplitude separately for each peak, the impact of thermogenic sweating can be greatly reduced.

In an embodiment, the processing unit can be adapted such that, for each of said skin conductance peaks, said normalized parameter is a normalized steepness of a portion of the respective skin conductance peak, in particular a normalized maximum rising edge slope of the respective skin conductance peak. It will be understood that this may also include that the normalized parameter is indicative of or determined based on a normalized amplitude of the respective skin conductance peak. An advantage of this embodiment can be that an efficient calculation can be provided. Further, by determining a normalized steepness separately for each peak, the impact of thermogenic sweating can be greatly reduced.

In a further refinement, the processing unit can be adapted such that the normalized steepness is determined based on a difference of a logarithm of a first value of the skin conductance signal trace and a logarithm of a second value of the skin conductance signal trace at the respective skin conductance peak. An advantage of this embodiment can be an efficient calculation. Optionally, only rising edge steepness, or in the alternative only falling edge steepness, may be used in this procedure. Optionally, in addition or in the alternative, the steepness can be averaged over a time period. This can mean averaging or downsampling a skin conductance signal trace acquired at a sampling rate of 160Hz to 1 Hz, or when the context is known averaging over a stressful episode that can stretch over several minutes. Advantages of this aspect may refer to improving accuracy and/or lowering the sensitivity for signal noise.

In an embodiment, the processing unit can be adapted such that determining the normalized parameter of the skin conductance peak comprises determining at least two points in said skin conductance peak, in particular in a raising edge of a skin conductance peak, and taking a relative, normalized measurement of a value of the skin conductance signal trace at the two points. Optionally, the approach according to an aspect of this disclosure may comprise the steps of extracting skin conductance (SC) peaks/ responses from the skin conductance signal trace; determining at least two points in the peak, in particular rising part of the peak; taking a (relative) normalized measurement of the two points; and processing the (relative) normalized measurements to determine psychogenic stress.

As used herein, a normalization or taking a normalized measurement can refer to performing a normalization so as to arrive at a unit-less normalized representation fo the measured quantity, here of the skin conductance. In contrast to such a normalization, simply subtracting two values leads to a difference that still has the same unit such as $\mu$S (microSiemens).

In an embodiment, the processing unit can be adapted to determine the normalized parameter of the skin conductance peak based on a peak value and an onset value of the skin conductance peak, wherein the peak value is indicative of a skin conductance level at a peak of said skin conductance peak and wherein the onset value is indicative of a skin conductance level at an onset of the skin conductance peak.

In a further refinement, the processing unit can be adapted to determine the normalized parameter of the skin conductance peak based on a ratio of a first and a second value, wherein the first value is determined based on a difference between said peak value and said onset value; and wherein said second value is said peak value or said onset value. For example, a normalized skin conductance peak amplitude, also referred to as normalized skin conductance response amplitude can be determined by:

$$SCR_{normalized\_amplitude} = \frac{peak\_level\_value - onset\_level\_value}{onset\_level\_value},$$

wherein the peak (level) value is indicative of a skin conductance level at a peak of said skin conductance peak and wherein the onset (level) value is indicative of a skin conductance level at an onset of the skin conductance peak.

In an embodiment, the processing unit can further be adapted to determine a thermogenic sweat level based on (a difference between) the obtained skin conductance signal trace and the determined psychogenic stress level. As described above, the skin conductance comprises contributions due the psychogenic and thermogenic sweating. Based on the determined psychogenic stress level and the acquired skin conductance signal trace, it is thus possible to derive an indication of the thermogenic sweating. In other words, by subtracting a psychogenic contribution from the overall sweat level, the thermogenic sweat level or at least an indication thereof can be determined. It shall be understood that this may not be limited to a subtraction of individual sample values in a strict mathematical sense. A contribution of psychogenic sweating may be determined and an overall sweat level may be separated into a contribution due to psychogenic sweating and a contribution due to thermogenic sweating.

In an embodiment, the device can further be configured to (a) obtain temperature information and/or motion information of the subject; and (b) to determine the psychogenic stress level of the subject based on said skin conductance signal trace and further based on said temperature information and/or motion information of the subject. Correspondingly, in addition or in the alternative to step (b), said temperature and/or motion information can offer additional information pertaining to occurrence of thermoregulation (rather than psychogenic sweating). Optionally, the processing unit can be adapted to evaluate a relationship or correlation between a thermogenic sweat level and temperature and/or physical activity. The device or system may comprise a temperature sensor for acquisition of said temperature information. The device or system may comprise a motion sensor such as (3D) accelerometer or gyroscope. The motion sensor can provide additional information pertaining to physical activity. Temperature and/or physical activity data (such as motion information) can give information on the cause of the observed thermoregulation. Optionally, quantification of thermogenic sweating can offer information on the perception of the ambience by the user, which can optionally be conveyed to climate control or a clothing advice application. Optionally, the device can be adapted to classify a type and/ or extent of thermogenic sweating (e.g. temperature-related or exercise-related thermogenic sweating). In a further refinement, based on the (extent of or information about) thermogenic sweating, the device may be adapted to estimate an extent of water loss. Based thereon an impact on hydration of the user can be determined. Hence, in addition to determining information about a psychogenic sweat level, the device may be adapted to provide information about a thermogenic sweat level (optionally including an indication of the extent of temperature-related and/or exercise-related thermogenic sweating). The device may further be adapted to provide a climate control signal based on said information. Optionally, the climate control signal may comprise clothing advice. The clothing advice may comprise an indication of how to adapt the user's clothing to reduce thermogenic sweating. Optionally, the device may be adapted to combine information about a thermogenic sweat level with information pertaining to physical activity; and to provide an advice for a (recommended) activity level based thereon. For example, people may want to prevent sweating, e.g. when cycling to work in the morning. Hence, the device may be adapted to provide a sweat warning it the thermogenic sweating exceeds or is about to exceed a predetermined threshold, in particular based on the physical activity level. On the other hand, a person may want to sweat as a result of their exercises, so that the device can be measured to which extent this goal is reached.

In an embodiment of the system, the sensor for acquiring the skin conductance signal trace can be adapted to acquire the skin conductance signal trace on a dorsal side of a wrist of the user. Correspondingly, the processing unit of the device can be adapted to process a skin conductance signal indicative of a skin conductance at a dorsal side of a wrist of the subject. In particular, the system can be adapted for measurement at a non-glabrous skin part, in particular at an upper side of the wrist or ankle of the user.

Optionally, the sensor may comprise one or more elongated electrodes that are arranged such that, in an as worn orientation, they are in line with muscle tendons of an upper (distal) side of the wrist of the user. An advantage of such a combination of shape an orientation can be that dropouts in the skin conductance signal caused by hand or wrist movements can be minimized or at least reduced. Dropouts can be indicative that (part of) the electrode is no longer in contact with the skin.

In an embodiment of the wearable device, the wearable device may comprise a wearable device housing and at least one electrode of the sensor for skin conductance measurement may be integrated in the housing of the wearable device (instead of for example being integrated in a wristband or even a separate electrodes connected via separate leads). Advantages of this embodiment can comprise easier system integration and/or better electrical contact since the electrode may, in an as worn orientation, lie on an upper side an arm or wrist of the user by gravity.

In an embodiment, the proposed system may further comprise and an interface, in particular a user interface, to convey stress response information to the user or an application or device that utilizes the stress response information or a to a healthcare professional or caregiver.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a system and a device for determining a psychogenic stress level of a user;
Fig. 2 shows an exemplary implementation of a system according to the present invention in form of a wearable device;
Fig. 3 shows a diagram of a skin conductance trace;
Fig. 4 shows a diagram of a skin conductance peak or skin conductance response;
Fig. 5 shows diagrams of a raw skin conductance trace signal, a filtered skin conductance trace signal and of skin conductance peaks identified therein;

Fig. 6 shows diagrams of a skin conductance trace signal, absolute rising edge heights and normalized rising edge heights extracts from said skin conductance signal trace;

Fig. 7 shows further diagrams of a skin conductance trace signal, absolute rising edge heights and normalized rising edge heights extracts from said skin conductance signal trace;

Fig. 8 shows diagrams of a skin conductance trace signal, determined stress level based on absolute rising edge heights and determined stress level based on normalized rising edge heights extracts from said skin conductance signal trace;

Fig. 9 shows a diagram of a skin conductance trace signal and a positive first order derivative or steepness of the absolute rising edge heights;

Fig. 10 shows a diagram of a skin conductance trace signal and a positive first order derivative of a logarithmic transformed skin conductance;

Fig. 11 shows a flow chart of an embodiment of a method for determining a psychogenic stress level of a user.

DETAILED DESCRIPTION OF THE EMBODIMENTS

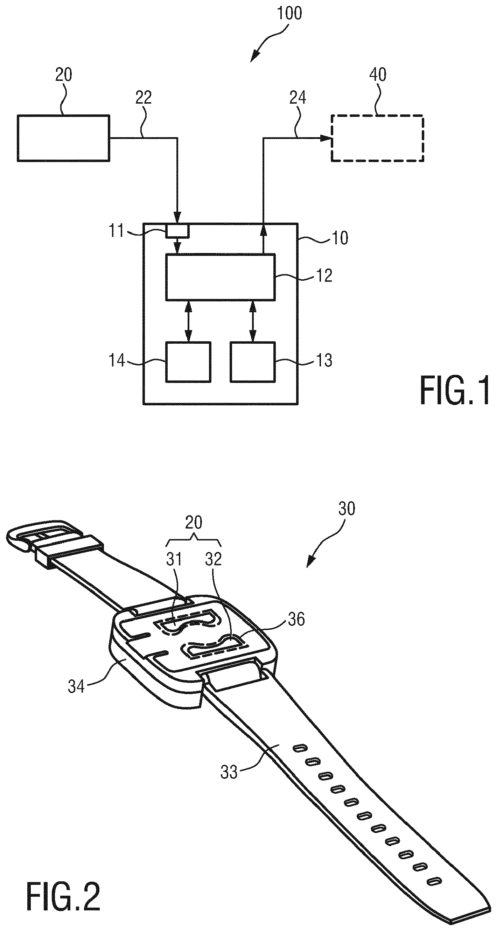

Fig. 1 shows a schematic diagram of exemplary embodiment of a system 100 for determining a stress level of a user. The system 100 comprises a sensor 20 for measuring a skin conductance signal of the user. The skin conductance signal measured by the sensor 20 over time forms a skin conductance signal trace 22 which is generally indicative of one or more measured stimulus responses corresponding to a neural stress response. The system 100 further comprises a device 10 for determining a stress level of the user.

The device 10 comprises an interface 11 for obtaining (i.e. receiving or retrieving from the sensor 20 or a (not shown) memory) a skin conductance signal trace 22 of the user and a processing unit 12 for processing the obtained skin conductance signal trace 22. The processing unit 12 can be any type of suitable processing unit or processor, such as for example a microprocessor/microcontroller, or embedded microcontroller but not limited thereto that is adapted accordingly. The interface 11 can be any kind of interface from obtaining data from the sensor 20 or a memory, e.g. a wireless or wired data interface or signal line. It will be understood that the sensor 20 and the device 10 can be part of the same device (e.g. wearable device or wristband) or can be implemented as or in separate devices.

The processing unit 12 can be adapted to perform the steps of identifying a plurality of skin conductance peaks in the skin conductance signal trace; determining, for each of said skin conductance peaks, a normalized parameter of said skin conductance peak, normalized based on a skin conductance value of the respective skin conductance peak; and determining a psychogenic stress level of the user based on said normalized parameters of said skin conductance peaks. Details of exemplary embodiments of the processing performed by the processing unit 12 will be explained below.

Optionally, as indicated by the dashed lines in Fig. 1, the system 100 can comprise an output unit 40 for outputting or rendering the determined stress level 24 to a user. The output unit 40 can be a human-machine-interface (HMI). For example, a display, an LED or a buzzer can be provided. Optionally, the output unit may be adapted to discretely indicate a stress level to the user. For example, a buzzer or vibration unit can be provided, which can discreetly signal a stress level increase. In addition or in the alternative, an optical indication can be provided. For example, the stress level may be communicated with a programmable LED, e.g. blue for the lowest level, followed by green, yellow, orange and lastly red for the highest stress level.

It will be understood that the output unit 40 and the device 10 can be part of the same device (e.g. wearable device or wristband) or can be implemented as or in separate devices. For example, the output unit 40 of the system 100 may be implemented by means of a smartphone or other information processing entity at the same or a remote location. Correspondingly, the processing unit 12 can also be implemented by means of a smartphone that is adapted to perform the afore-mentioned functionality for example by running a corresponding application or another suitable computing device running the corresponding software.

The system 100 may further comprise a memory 13 for storing program code means for causing the processor 12 to carry out the steps of the method as described herein. Details will be explained below. The memory 13 can be part of the device 10 or can be an external memory. The memory can be any suitable memory such as for example a memory register or RAM (random access memory). Advantageously, a non-volatile memory can be used, for example a microSD flash card. It will be understood that the memory 13 and the processing unit 12 can be part of the same device (e.g. wearable device or wristband) or can be implemented as or in separate devices.

Fig. 2 shows an embodiment of a wearable device 30 wearable by user. In this embodiment, the wearable device 30 is implemented in the form of a smart watch. The smart watch comprises a wristband 33 and a casing 34. The wristband 33 can loop around the wrist of the user. It will be understood that a wearable device could also be worn around another suitable part of the body such as the ankle foot or hand or may be adapted for attachment to other parts of the body, e.g. in the form of a patch.

The wearable device 30 can comprise the proposed system 100 for determining a stress level of a user. In this way a corresponding system 100 can be provided in an unobtrusive and wearable format. Alternatively, the wearable device 30 may only comprise the sensor 20, in this embodiment a skin conductance sensor 20. The device 10 of the system 100 may be located at the remote location or implemented in a remote device (e.g. a remote computer, smartphone or patient monitor).

The skin conductance sensor 20 may comprise a first and a second skin conductance electrode 31, 32 in combination with a skin conductance measuring unit (not shown). In the embodiment of Fig. 2, two skin conductance electrodes 31, 32 are integrated into the casing of the wearable device, however is also possible to integrate them for example into the wristband 33 And thus contact the underside of the wrist. The skin conductance electrodes 31, 32 can be arranged so as to contact the upper side of the wrist when the wearable device 30 is worn by the user. Exemplary implementations of a wearable device comprising a skin conductance sensor are the Philips discreet tension indicator DTI-4 or DTI-5.

The skin conductance measurement electrodes 31, 32 can be arranged for acquiring the skin conductance signal trace at a dorsal side of a wrist of the user, i.e. at an upper side of the wrist of the user in an as-worn orientation of the wearable device 30. As shown in Fig. 2, the elongated electrodes are arranged such that, in an as worn orientation, they are in line with muscle tendons of an upper (distal) side of the wrist of the user. An advantage of such a combination of shape and orientation can be that dropouts in the skin conductance signal caused by hand or wrist movements can be minimized or at least reduced. Dropouts can be indicative that (part of) the electrode is no longer in contact with the skin.

Optionally, the device 10, in particular the wearable device 30 can be fitted with a micro climate enhancement electrode surrounding inlay 36 such as described in US 9,706,942 B2, the contents of which are incorporated herein by reference. The micro climate inlay can be shaped such that it is level with the skin conductance electrodes, thus minimizing skin deformation, thus avoiding skin irritation. The micro climate inlay can also be referred to as a micro climate flap or micro climate enhancing rubber.

The skin conductance sensor 20 is adapted to measure the skin conductance of the user 2 between the skin conductance electrodes 31, 32. For this purpose, the skin conductance measuring sensor may comprise a voltage generator for applying a voltage between the at least two skin conductance electrodes, a sensing unit for sensing a current between the at least two electrodes, and/or a calculating unit for calculating the skin conductance based on the sensed current. The measured skin conductance over time forms the skin conductance signal trace. The skin conductance signal trace (or data) may for example be stored in a memory of the wearable device 30, or may be transmitted (wirelessly or through a wire or signal line) to an external unit.

The skin conductance measuring sensor 20 and/or the device 10 (as shown in Fig. 1) may be integrated into the casing 34 of the wearable device 30. The wearable device 30 can further comprise a transmitter for transmitting data over a wireless or wired communication link, such as the measurement data and/or the determined stress level 24 of the user. However, it will be understood that the device 10 or processing unit 12 can also be implemented as or in separate parts or devices and that the wearable device 30 then transmits the skin conductance data to the separate part or device via the transmitter.

Advantageously, the system 100 may also comprise an output unit 40 for outputting the stress level of the user. The output unit 40 may be a separate device or may be integrated into, for example, the wearable device 30 comprising the sensor 20 in form of a smart watch. Furthermore, an external output unit 40, for example a smartphone, tablet or PC running a corresponding application, may be used and coupled to the device 10 or wearable device 30.

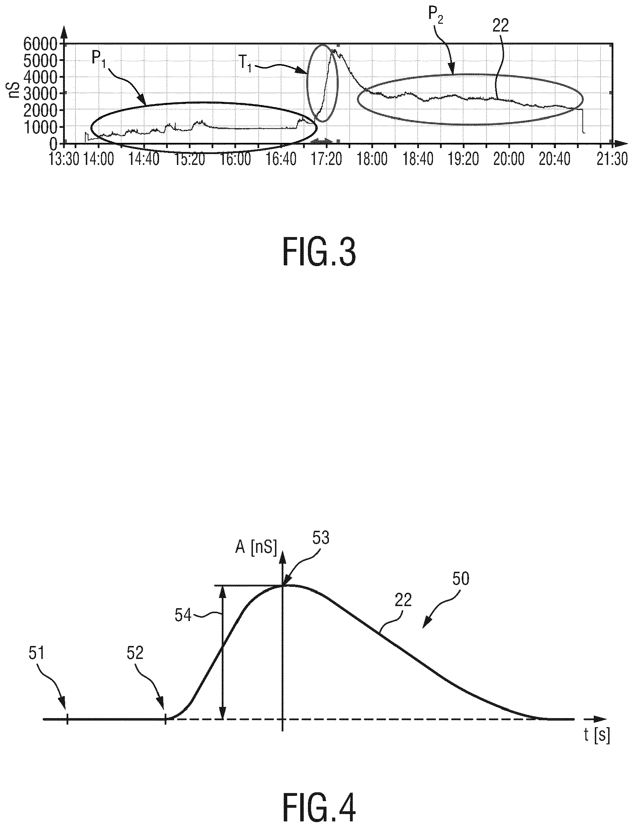

In the following, details of the proposed approach will be explained. Fig. 3 shows an exemplary skin conductance signal trace 22. The horizontal axis denotes the time of the day. The vertical axis denotes the (raw) measured skin conductance in nS (nanoSiemens). In the given example, the skin conductance signal trace 22 comprises three main portions indicated by PI, T1, and P2. The first period P1 (first period of psychogenic sweating) from about 13:50h to 17:10h denotes a time of psychogenic sweating, wherein the user was in a temperature-controlled environment and concentrated on a mental task but non engaging in physical activity. Sweating during this period P1 can be attributed to psychogenic sweating. The second period T1 (first period of thermogenic sweating) from about 17:10h to 17:30h denotes a period by thermogenic sweating, wherein the user engages in heavy physical activity (cycling for catching the train at 17:27h). Sweating during this period P2 can be attributed to psychogenic sweating. The third period of time P2 (second period of psychogenic sweating) again denotes a time in a temperature controlled environment without physical activity. Skin conductance responses in this time period may again be attributed to psychogenic sweating.

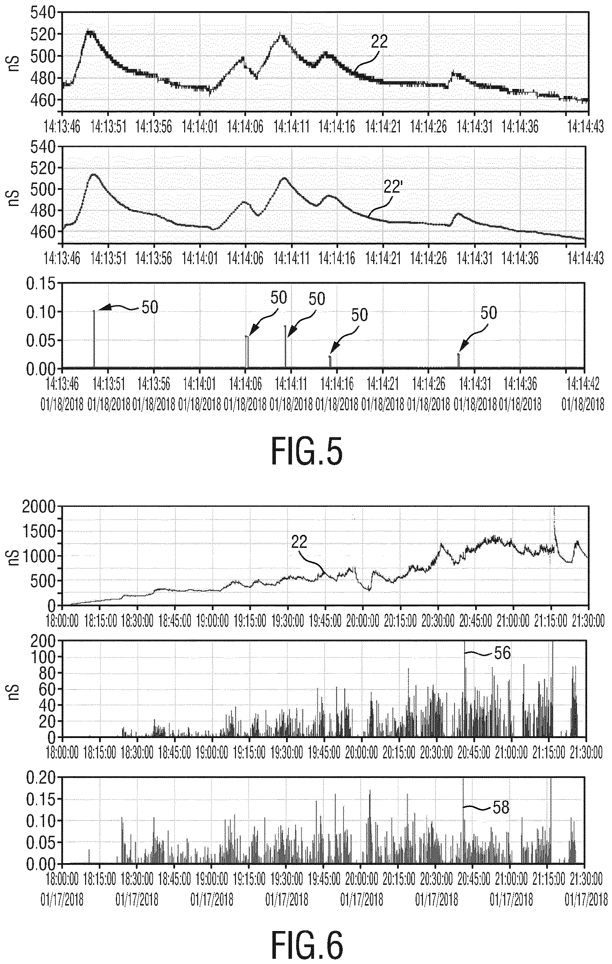

Fig. 5 shows an individual skin conductance response or skin conductance peak 50. The curve in Fig. 5 can be seen as an (extremely) magnified portion of the skin conductance signal trace 22 in Fig. 3. The horizontal axis again denotes the time t, whereas the vertical axis denotes the skin conductance in [nS]. The graph in Fig. 4 spans about 15 seconds, and the graph in Fig. 3 spans about 8 hours. As used herein, a skin conductance peak 50 does not only refer to the maximum point but rather refers to a portion of the respective skin conductance response signal 22. A psychogenic stimulus may occur at the moment denoted by 51. In response to said stimulus, with a slight delay, the skin conductance starts to increase at the onset denoted by 52 until the skin conductance peak 50 reaches its maximum value at 53. The delay can for example be about 1 second for the wrist and about 2 seconds for the ankle. This can be attributed to the signal velocity along the sympathetic nerve. The difference 54 between the skin conductance level at the onset 52 and the skin conductance level at the maximum 53 or peak provides the skin conductance peak or skin conductance response amplitudes (SCR$_{amplitude}$).

Fig. 5 illustrates signals during different stages of the signal processing. The horizontal axis in the graphs denotes the time, whereas the vertical axis denotes an amplitude. As shown in the upper graph in Fig. 5, the skin conductance signal trace 22 may be measured by the device at a sampling frequency between 40 and 160 Hz. As shown in the middle graph in Fig. 5, this raw signal can optionally be low-pass filtered to yield a smooth down-sampled signal 22' having a sampling rate of, for example, 10 Hz. Subsequently, a plurality of skin conductance peaks 50 can be identified in the skin conductance signal trace. For example, rising edges in the filtered signal can be detected by zero-crossings of the first derivative of the (optionally filtered or preprocessed) skin conductance signal 22.

As an optional further processing step, additional processing and filtering steps can be applied. For example short glitches or slow signal drifts may be eliminated. In the given example, only rising edges that have a duration longer than 0.8 seconds and shorter than 3.0 seconds are attributed to skin conductance responses (SCR). Optionally, it is also possible to count every rising edge, even those lasting longer than 3 seconds or only rising edges lasting longer than a predetermined value, for example longer than 0.8 seconds. It has been found that when there are strong emotions, the skin conductance level may rise so fast that no or only too few zero crossings of the first order derivative are observed (e.g., just ripples in the rising edge). Not taking this into account may lead to missing strong emotions. Optionally, the processing device can thus be configured to determine strong emotional responses based on ripples in the rising edge of (a first derivative of) a skin conductance signal trace. It should be noted that skin conductance responses or skin conductance peaks can be identified using known techniques, as for example described in the aforementioned standard textbooks.

However, in contrast to the generally accepted procedure, the inventors have recognized that by determining, for each of said skin conductance peaks, a normalized parameter of said skin conductance peak, normalized based on a skin conductance value of the respective skin conductance peak, the impact of thermogenic sweating can be reduced, thereby providing a more meaningful indication of psychogenic sweating. For example, a normalized relative skin conductance peak amplitude, also referred to as normalized skin conductance response amplitude can be determined by:

$$SCR_{normalized\_amplitude} = \frac{peak\_level\_value - onset\_level\_value}{onset\_level\_value},$$

wherein the peak (level) value is indicative of a skin conductance level at a peak (see 53 in Fig. 4) of said skin conductance peak and wherein the onset (level) value is indicative of a skin conductance level at the onset (see 52 in Fig. 4) of a skin conductance peak.

The result of this calculation is shown in the lower graph in Fig. 5, wherein the normalized skin conductance response amplitudes, as exemplary a normalized parameters of said skin conductance peak, normalized based on a skin conductance value of the respective skin conductance peak, are denoted by reference numeral 50.

The normalized parameter of the skin conductance peak, here SCR$_{normalized\_amplitude}$ can be a dimensionless number that represents the normalized height of the respective skin conductance response. More generally speaking, determining the normalized parameter can comprise scaling a first value, for example the peak level value, of the skin conductance signal trace at the respective skin conductance peak based on a second value, for example the onset level value, of the skin conductance signal trace at the respective skin conductance peak. It should be noted that the proposed approach differs from what is normally used in the practice of extracting meaningful data from a skin conductance trace. In the aforementioned standard textbook Techniques in Psychophysiology the standard method for obtaining the SCRamplitude provides an absolute amplitude, that is determined by

$$SCR_{absolute\_amplitude} = peak\_level\_value - onset\_level\_value$$

wherein the peak (level) value is indicative of a skin conductance level at a peak (see 53 in Fig. 4) of said skin conductance peak and wherein the onset (level) value is indicative of a skin conductance level at an onset (see 52 in Fig. 4) of the skin conductance peak. In other words, in contrast to the solution proposed herein, the standard method for measuring the amplitude of the skin conductance response only takes the skin conductance level at the top and deducts the skin conductance level at the onset, thus yielding a number with the same dimension as the skin conductance (usually micro Siemens). This (conventional) amplitude can be referred to as absolute skin conductance response amplitude, or SCR$_{absolute\_amplitude}$.

In Fig.6, the top graph shows a skin conductance signal trace 22, acquired with a Philips discreet tension indicator DTI-5 covering a time frame of 3.5 hours. The horizontal axis denotes the time. The vertical axis denotes the skin conductance in nS (nanoSiemens). The level of the skin conductance 22 gradually rises in this time frame. The middle graph in Fig. 6 represents the non-normalized $SCR_{absolute\_amplitude}$ 56 for each of a plurality of skin conductance peaks and the bottom graph represents the normalized $SCR_{normalized\_amplitude}$ 58 for each of a plurality of skin conductance peaks that were extracted from the skin conductance trace 22. The $SCR_{absolute\_amplitude}$ 56 correlates with the average level of the skin conductance, whereas the $SCR_{normalized\_amplitude}$ 58 does not show this correlation. Nonetheless, even though by the proposed peak-to-peak normalization, the information content is reduced, it has been found that the proposed normalization may eliminate or at least reduce thermogenic influences from the extraction of a (psychogenic) stress level from skin conductance data.

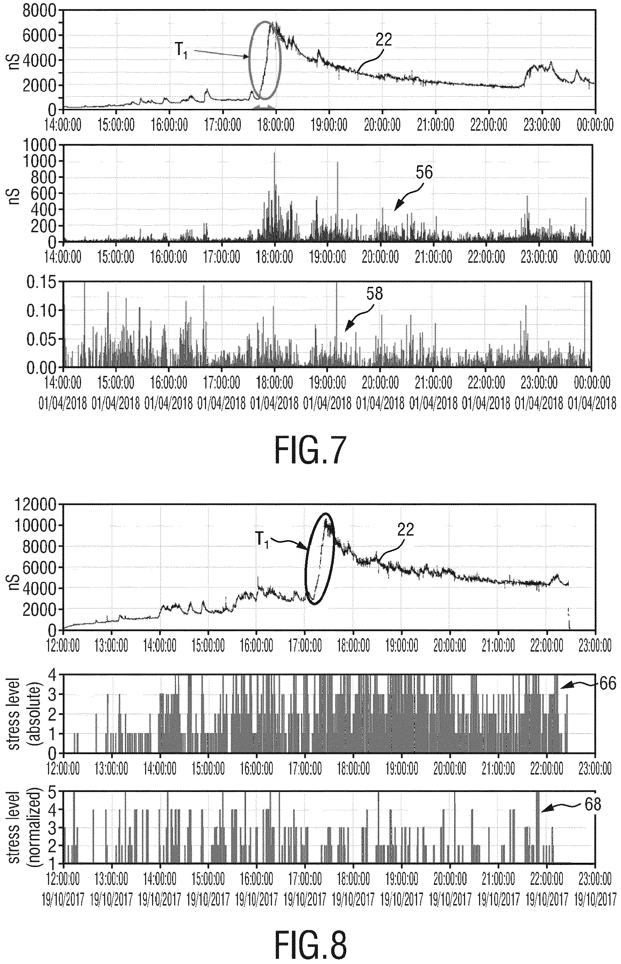

In Fig. 7 this approach is shown for a skin conductance trace 22 that contains thermogenic sweating influences that can be attributed to fast cycling to catch a train in time frame $T_1$, as shown in the upper graph in Fig. 7. The middle graph in Fig. 7 again represents the non-normalized $SCR_{absolute\_amplitude}$ 56 for each of a plurality of skin conductance peaks and the bottom graph represents the normalized $SCR_{normalized\_amplitude}$ 58 for each of a plurality of skin conductance peaks that were extracted from the skin conductance trace 22. It can be seen clearly that the non-normalized $SCR_{absolute\_amplitude}$ 56 increase caused by the cycling activity has negligible influence on the normalized $SCR_{normalized\_amplitude}$ 58 according to the solution proposed herein. Hence, an influence of the skin conductance level variation of the quantification of the skin conductance response amplitude due to thermogenic sweating can be eliminated or at least reduced.

From the middle and bottom graphs in Fig. 7, it also becomes clear that in the two time periods before and after bicycling, the height of the $SCR_{normalized\_amplitude}$ peaks is comparable, truthfully reflecting that their levels of stress are comparable as well. Thus the $SCR_{normalized\_amplitude}$ measure may ensure that psychogenic peaks are given equal weight irrespective of the underlying skin conductance level, which might have greatly increased due to thermogenic sweating, especially at the outside of the wrist.

The conversion of the normalized skin conductance amplitude values to a stress level can be performed using known techniques. For example, a sum of (normalized) rising edge amplitudes per predetermined time interval can be evaluated. Based on a histogram of said sums of (normalized) rising edge amplitudes, different stress levels can be determined and thus user classified or categorized to a corresponding stress level. In the given non-limiting example, five different stress levels are provided, as shown in Fig. 8.

In Fig. 8, the top graph shows a portion of the skin conductance signal trace 22 of the top graph in Fig. 7. The graph again includes a time frame $T_1$ of physical activity, here fast cycling to catch a train. The middle graph in Fig. 8 shows a stress level 66 of the user determined based on a sum of conventional non-normalized rising edge amplitudes (cf. Fig. 7, middle graph). On the other hand, the bottom graph in Fig. 8 shows a stress level 68 of the user determined based on as sum of normalized rising edge amplitudes (cf. Fig. 7, bottom graph). As can be seen from a comparison of the middle and bottom graphs in Fig. 8, the impact of thermogenic sweating that is present during the physical activity from 17:20h onwards. It should be noted that a reduction of mental stress when cycling to the train station also accurately reflects a perceived mental stress level of the user during the measurement.

Referring again to Fig. 7, the inventors have recognized that, further to evaluating the normalized parameters of said skin conductance peaks, as represented in the bottom graph of Fig. 7, the processing unit can also be configured to evaluate a difference between the non-normalized parameters and the normalized parameters. For example, a difference between the non-normalized $SCR_{absolute\_amplitude}$ 56 for each of a plurality of skin conductance peaks and the normalized $SCR_{normalized\_amplitude}$ 58 for each of a plurality of skin conductance peaks that were extracted from the skin conductance trace 22 can be evaluated. Since the non-normalized $SCR_{absolute\_amplitude}$ 56 comprises contributions due to thermogenic sweating and psychogenic sweating and the normalized $SCR_{normalized\_amplitude}$ 58, as a first order approximation, reflects contributions due to psychogenic sweating (only), the difference between the two may yield the effects of thermogenic sweating (only). This can itself be valuable information.

Moreover, as indicated in Fig. 1 the device 10 may optionally be provided with a temperature and/or motion sensor 14. Temperature or physical activity data can give information on the cause of the observed thermoregulation. In particular, quantification of thermogenic sweating may offer information on the perception (and impact) of the ambient by (on) the user. Such information may be conveyed to climate control and/or a clothing advice application.

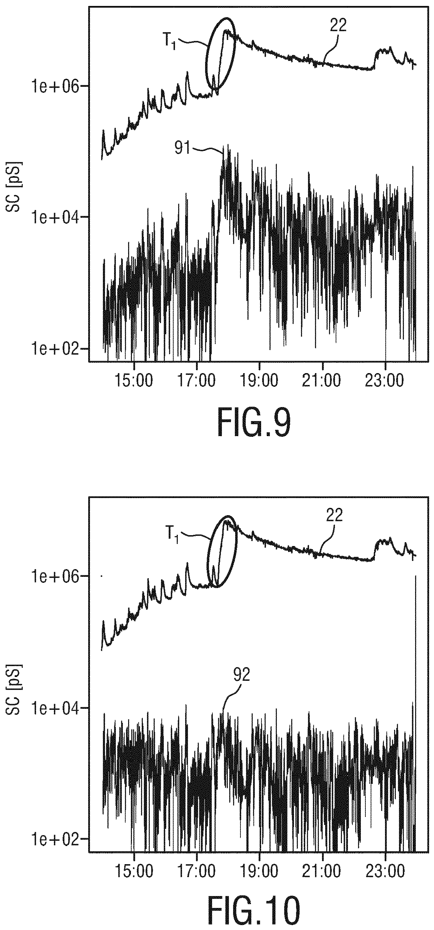

Fig. 9 and 10 show graphs regarding a further embodiment of processing skin conductance data for determining a normalized parameter. Fig. 9 shows a diagram of a skin conductance trace signal (SC) 22 (in [pS] or picoSiemens) and a positive first order derivative or steepness curve 91 of the absolute rising edge heights (in [pS/s]). In Fig. 9, the curve 91 denotes the steepness of the absolute rising edge heights (in [ps/s]) that can be calculated by

$$steepness_{absolute} = SC_{i+1} - SC_i$$

wherein $SC_i$ is the sample value at point i, and $SC_{i+1}$ is the sample value at point i+1. On logarithmic scale, this can be rewritten as:

$$steepness_{absolute,\log} = \left(10\log\left(SC_{i+1} - SC_i\right)\right)\cdot f$$

The curve 91 shown in Fig. 9 shows a moving average over the last 30 samples. In the given example, zero and negative steepness values have been discarded. Nonetheless, as can be seen from Fig. 9, the time interval $T_1$ of physical arousal (here running or cycling to catch a train) shows a strong contribution to the curve 91. Optionally, a maximum rising edge slope of each of a plurality of skin conductance peaks can be identified. Thereby, by only looking at the maximum of the rising edge slope per peak, the amount of data to be stored may be reduced. The terms steepness and slope may be used interchangeably.

Fig. 10 shows a diagram of a skin conductance trace signal 22 (in [pS] or picoSiemens) and a positive first order derivative of a logarithmic transformed skin conductance 92 (in [log10(pS)/s]), as will be described below. It has been found that the thermogenic contribution to the skin conductance data 22 can also be removed by using a measure for the steepness of the rising edges. The steepness can be calculated by taking the difference of logarithmic values of skin conductance measurement i and measurement i+1. Optionally, the value can be multiplied with the sampling:

$$steepness_{normalized,\log} = \left(10\log\left(SC_i\right) - 10\log\left(SC_{i+1}\right)\right)\cdot f$$

wherein $SC_i$ denotes a sample value of the skin conductance signal trace at sample i; $SC_{i+1}$ denotes a sample value of the skin conductance signal trace at a subsequent sample i+1; and f denotes the sampling frequency. The sampling frequency is optional in the aforementioned formulae. Hence, a (positive) first order derivative of the skin conductance (SC) signal 22 after conversion to a logarithmic scale, e.g. by log10(SC), can be used as an estimator for arousal. It has been found that this estimation may be less sensitive for one or more of the following effects: thermogenic heating, building up micro-climate after mounting, or intense manual labor or exercise. For such a log-calculation, all negative values can be set to zero, leaving only the rising edges of the skin conductance trace. If the logarithm is not used the thermogenic effects are clearly visible in the steepness curve 91 as is shown in Fig. 9. By calculating steepness according to the formula shown above (i.e. by taking a difference of the logarithms) the thermogenic effects are no longer visible in the steepness curve 92 as is shown in Fig. 10. Instead of steepness, it can also be called the first time derivative. Optionally, a normalized maximum rising edge slope of each of a plurality of respective skin conductance peaks can be identified and used for further processing.

It should be noted that the proposed calculation may again be considered as obtaining a normalized parameter of said skin conductance peak, normalized based on a skin conductance value of the respective skin conductance peak. The aforementioned equation may also be rewritten as:

$$steepness = \left(10\log\left(\frac{SC_i}{SC_{i+1}}\right)\right)\cdot f$$

It will be understood that in the aforementioned equations, the sample $SC_{i+1}$ may be replaced by $SC_{i-i}$ and vice versa.

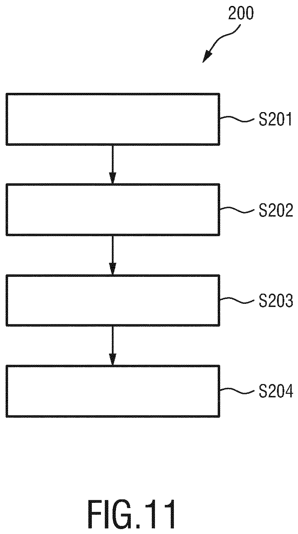

Fig. 11 shows a schematic flow chart of a method for determining a stress level of a user. The method 200 comprises a first step S201 obtaining a skin conductance signal trace (data) of the user. For example, the skin conductance signal trace can be obtained directly as output data from a sensor (or preprocessing unit) or can be retrieved from storage means, for example, a memory or a hospital information system (HIS) or electronic health record (EHR) of the user.

In the next step S202 a plurality of skin conductance peaks are identified in the skin conductance signal trace. In step S203, for each of said skin conductance peaks, a normalized parameter of said skin conductance peak is determined, wherein the respective parameter is normalized based on a skin conductance value of the respective skin conductance peak. For example, a normalized amplitude or normalized edge steepness of the skin conductance peak may be determined.

In step S204, a psychogenic stress level of the user can be determined based on said normalized parameters of said skin conductance peaks.

In conclusion, the proposed solution may assist in quantifying the stress responses that are linked to psychogenic sweating which occur in a skin conductance trace. Advantageously, such a determination may be impervious or less susceptible to skin conductance changes such as those caused by physical activity or climate induced thermogenic sweating.

Exemplary applications of the propose solution may include, but are not limited to stress response measurement at non-glabrous (hairy) skin locations; climate control for vehicles or rooms based on the measurement of thermoregulation, clothing advice based on the measurement of thermoregulation.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (10) for determining a stress level (68) of a user, said device comprising:

   - an interface (11) configured to obtain a skin conductance signal trace (22) of the user;
   - a processing unit (12) configured to process the obtained skin conductance signal trace by:
   - identifying a plurality of skin conductance peaks (50) in the skin conductance signal trace;
   - determining, for each of said skin conductance peaks, a normalized parameter (58) of said skin conductance peak, normalized based on a skin conductance value (52, 53) of the respective skin conductance peak (50); and
   - determining a psychogenic stress level (68) of the user based on said normalized parameters of said skin conductance peaks.

2. Device as claimed in claim 1, wherein, for each of said skin conductance peaks (50), determining the normalized parameter (58) comprises scaling a first value (52, 53) of the skin conductance signal trace at the respective skin conductance peak based on a second value (52, 53) of the skin conductance signal trace at the respective skin conductance peak.

3. Device as claimed in any of the preceding claims, wherein, for each of said skin conductance peaks (50), said normalized parameter is a normalized amplitude of the respective skin conductance peak.

4. Device as claimed in claim 1 or 2, wherein, for each of said skin conductance peaks (50), said normalized parameter (58) is a normalized steepness (92) of a portion of the respective skin conductance peak.

5. Device as claimed in claim 4, wherein the normalized steepness (92) is determined based on a difference of a logarithm of a first value of the skin conductance signal trace and a logarithm of a second value of the skin conductance signal trace at the respective skin conductance peak (50).

6. Device as claimed in any of the preceding claims, wherein determining the normalized parameter (58) of the skin conductance peak (50) comprises determining at least two points (52, 53) in said skin conductance peak, in particular in a raising edge of a skin conductance peak, and taking a relative measurement of a value of the skin conductance signal trace (22) at the two points.

7. Device as claimed in any of the preceding claims, wherein the normalized parameter of the skin conductance peak is determined based on a peak value (53) and an onset value (52) of the skin conductance peak, wherein the peak value is indicative of a skin conductance level at a peak of said skin conductance peak and wherein the onset value is indicative of a skin conductance level at an onset of the skin conductance peak.

8. Device as claimed in claim 7, wherein the normalized parameter (58) of the skin conductance peak is determined based on a ratio of a first and a second value, wherein the first value is determined based on a difference between said peak value (53) and said onset value (52); and wherein said second value is said peak value or said onset value.

9. Device as claimed in any of the preceding claims, wherein the processing unit (12) is further adapted to determine a thermogenic sweat level based on the obtained skin conductance signal trace (22) and the determined psychogenic stress level.

10. Device as claimed in any of the preceding claims, wherein the device (10) is further configured to

    - obtain temperature information and/or motion information of the subject; and
    - to determine the psychogenic stress level (68) of the subject based on said skin conductance signal trace (22) and further based on said temperature information and/or motion information of the subject.

11. System (100) for determining a stress level (68) of a user, said system comprising:

    - a sensor (20) for acquiring skin conductance signal trace (22) from a user; and
    - a device (10) as claimed in any of the preceding claims.

12. System as claimed in claim 11, wherein the sensor (20) is adapted to acquire the skin conductance signal trace (22) on a dorsal side of a wrist of the user.

13. Wearable device (30) wearable by a user, the wearable device comprising the system (100) as claimed in claim 11 or 12.

14. Method (200) for determining a stress level of a user, the method comprising:

    - obtaining a skin conductance signal trace (22) of the user (S201);
    - identifying a plurality of skin conductance peaks (50) in the skin conductance signal trace (S202);
    - determining, for each of said skin conductance peaks, a normalized parameter (58) of said skin conductance peak, normalized based on a skin conductance value (52, 53) of the respective skin conductance peak (S203); and
    - determining a psychogenic stress level (68) of the user based on said normalized parameters of said skin conductance peaks (S204).

15. Computer program comprising program code means for causing a computer to carry out the steps of the method (200) as claimed in claim 14 when said computer program is carried out on a computer.

100

20

22

24

40

11

10

12

14

13

## FIG.1

30

20

31 32

36

34

33

## FIG.2

FIG.3

FIG.4

FIG.5

FIG.6

FIG.7

FIG.8

FIG.9

FIG.10

200

S201

S202

S203

S204

FIG.11

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2016/262690 A1 (CHEN TSAN-JIEH [TW] ET AL) 15 September 2016 (2016-09-15)<br>* paragraph [0026] *<br>* paragraph [0040] *<br>* paragraph [0057]; figures 10A, 10B *<br>----- | 1-4,6,7, 9-15<br>5,8 | INV.<br>G16H50/30<br>A61B5/00<br>G16H50/20<br>A61B5/053 |
| X<br>A | WO 2018/015308 A1 (KONINKLIJKE PHILIPS NV [NL]) 25 January 2018 (2018-01-25)<br>* page 2, lines 27-29; figure 2 *<br>----- | 1-4,6,7, 9,11-15<br>5,8,10 | |
| X<br>A | US 2010/022852 A1 (WESTERINK JOANNE HENRIETTE DESIREE MONIQUE [NL] ET AL) 28 January 2010 (2010-01-28)<br>* paragraph [0017]; figure 1 *<br>----- | 1-4,6,7, 9,11-15<br>5,8,10 | |

TECHNICAL FIELDS
SEARCHED      (IPC)

G16H
A61B

The present search report has been drawn up for all claims

| Place of search | Date of completion of the search | Examiner |
|---|---|---|
| Munich | 10 January 2019 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................................
& : member of the same patent family, corresponding
    document

EPO FORM 1503 03.82 (P04C01)

**EP 3 594 963 A1**

## ANNEX TO THE EUROPEAN SEARCH REPORT
## ON EUROPEAN PATENT APPLICATION NO.

EP 18 18 2894

10-01-2019

| Patent document cited in search report | Publication date | Patent family member(s) | | Publication date |
|---|---|---|---|---|
| US 2016262690 A1 | 15-09-2016 | CN | 105962911 A | 28-09-2016 |
| | | US | 2016262690 A1 | 15-09-2016 |
| WO 2018015308 A1 | 25-01-2018 | NONE | | |
| US 2010022852 A1 | 28-01-2010 | CN | 101610716 A | 23-12-2009 |
| | | EP | 2120701 A1 | 25-11-2009 |
| | | US | 2010022852 A1 | 28-01-2010 |
| | | WO | 2008099320 A1 | 21-08-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9962104 B2

- US 9706942 B2

**Non-patent literature cited in the description**

- Thermogenic and psychogenic recruitment of human eccrine sweat glands: Variations between glabrous and non-glabrous skin surfaces. **MACHADO-MOREIRA et al.** Journal of Thermal Biology. Elsevier, 2017

- Techniques in Psychophysiology. John Wiley & Sons Ltd, 1980
- Electrodermal Activity'' as well as in ''Electrodermal activity. Springer Verlag, 2012